# EUROPEAN PATENT SPECIFICATION

(11) **EP 1 349 515 B1**
(45) Date of publication and mention of the grant of the patent: **08.10.2008**
(21) Application number: 01271920.9
(22) Date of filing: 14.12.2001
(51) Int. Cl.: A61F 2/06

(54) **Endovascular prosthesis delivery system**
System zur einführung einer Endovaslulären Prothese
Système de délivrance d'endoprothèse vasculaire

(30) Priority: 15.12.2000 US 255398 P
(43) Date of publication of application: 08.10.2003
(73) Proprietor: Ricci, Donald R., Vancouver British Columbia V6R 1M9 (CA); Penn, Ian M., Vancouver, British Columbia V6R 1E4 (CA); Shukov, George A., Los Altos Hill, CA 94022 (US); Marotta, Thomas A., Etobicoke, Ontario M9A 2A6 (CA)
(72) Inventor: RICCI, Donald, R., Vancouver, British Columbia V6R 1M9 (CA); MAROTTA, Thomas, A., Etobicoke, Ontario M9A 2A6 (CA); BOURNE, Gyasi, Vancouver, British Columbia V6Z 1L4 (CA); MARKO, Alexei, Vancouver, British Columbia V5Y 1W9 (CA); MCDOUGALL, Ian, Vancouver, British Columbia V6H 3Z6 (CA); HONG, Elliot, Vancouver, British Columbia V6H 3Z6 (CA)
(74) Representative: Hirsch & Associés
(86) International application number: PCT/CA2001/001685
(87) International publication number: WO 2002/051336

(56) References cited:
- US-A- 5 484 449
- US-A- 5 578 009
- US-A- 5 634 902
- US-A- 5 662 702
- US-A- 5 846 259
- US-A- 6 139 564

## Description

The present invention relates to a catheter for endovascular delivery of a device and a catheterization kit.

### BACKGROUND ART

As is known in the art, an aneurysm is an abnormal bulging outward in the wall of an artery. In some cases, the bulging may be in the form of a smooth bulge outward in all directions from the artery - this is known as a "fusiform aneurysm". In other cases, the bulging may be in the form of a sac arising from one side of the artery - this is known as a "saccular aneurysm".

While aneurysms can occur in any artery of the body, it is only those which occur in the brain which lead to the occurrence of a stroke. Most saccular aneurysms which occur in the brain have a neck which extends from the cerebral blood vessel and broadens into a pouch which projects away from the vessel.

The problems caused by such aneurysms can occur in several different ways. For example, if the aneurysm ruptures, blood enters the brain or the subarachnoid space (i.e., the space closely surrounding the brain) - the latter is known as aneurysmal subarachnoid hemorrhage. This followed by one or more of the following symptoms: nausea, vomiting, double vision, neck stiffness and loss of consciousness. Aneurysmal subarachnoid hemorrhage is an emergency medical condition requiring immediate treatment. Indeed, 10-15% of patients with the condition die before reaching the hospital for treatment. More than 50% of patients with the condition will die within the first thirty days after the hemorrhage. Of those patients who survive, approximately half will suffer a permanent stroke. It is typical for such a stroke to occur one to two weeks after the hemorrhage itself from vasospasm in cerebral vessels induced by the subarachnoid hemorrhage. Aneurysms also can cause problems which are not related to bleeding although this is less common. For example, an aneurysm can form a blood clot within itself which can break away from the aneurysm and be carried downstream where it has the potential to obstruct an arterial branch causing a stroke. Further, the aneurysm can also press against nerves (this has the potential of resulting in paralysis or abnormal sensation of one eye or of the face) or the adjacent brain (this has the potential of resulting in seizures).

Given the potentially fatal consequences of the aneurysms, particularly brain aneurysms, the art has addressed treatment of aneurysms using various approaches.

Generally, aneurysms may be treated from outside the blood vessels using surgical techniques or from the inside using endovascular techniques (the latter falls under the broad heading of interventional (i.e., non-surgical) techniques).

Surgical techniques usually involve a craniotomy requiring creation of an opening in the skull of the patient through which the surgeon can insert instruments to operate directly on the brain. In one approach, the brain is retracted to expose the vessels from which the aneurysm arises and then the surgeon places a clip across the neck of the aneurysm thereby preventing arterial blood from entering the aneurysm. If there is a clot in the aneurysm, the clip also prevents the clot from entering the artery and obviates the occurrence of a stroke. Upon correct placement of the clip the aneurysm will be obliterated in a matter of minutes. Surgical techniques are the most common treatment for aneurysms. Unfortunately, surgical techniques for treating these conditions are regarded as major surgery involving high risk to the patient and necessitate that the patient have strength even to have a chance to survive the procedure.

As discussed above, endovascular techniques are non-surgical techniques and are typically performed in an angiography suite using a catheter delivery system. Specifically, known endovascular techniques involve using the catheter delivery system to pack the aneurysm with a material which prevents arterial blood from entering the aneurysm - this technique is broadly known as embolization. One example of such an approach is the Guglielmi Detachable Coil which involves intra-aneurysmal occlusion of the aneurysm via a system which utilizes a platinum coil attached to a stainless steel delivery wire and electrolytic detachment. Thus, once the platinum coil has been placed in the aneurysm, it is detached from the stainless steel delivery wire by electrolytic dissolution. Specifically, the patient's blood and the saline infusate act as the conductive solutions. The anode is the stainless steel delivery wire and the cathode is the ground needle which is placed in the patient's groin. Once current is transmitted through the stainless steel delivery wire, electrolytic dissolution will occur in the uninsulated section of the stainless steel detachment zone just proximal to the platinum coil (the platinum coil is of course unaffected by electrolysis). Other approaches involve the use of materials such as cellulose acetate polymer to fill the aneurysm sac. While these endovascular approaches are an advance in the art, they are disadvantageous. Specifically, the risks of these endovascular approaches include rupturing the aneurysm during the procedure or causing a stroke due to distal embolization of the device or clot from the aneurysm. Additionally, concern exists regarding the long term results of endovascular aneurysm obliteration using these techniques. Specifically, there is evidence of intra-aneurysmal rearrangement of the packing material and reappearance of the aneurysm on follow-up angiography.

One particular type of brain aneurysm which has proven to be very difficult to treat, particularly using the surgical clipping or endovascular embolization techniques discussed above occurs at the distal basilar artery. This type of aneurysm is a weak outpouching, usually located at the terminal bifurcation of the basilar artery. Successful treatment of this type of aneurysm is very difficult due, at least in part, to the imperative requirement that all the brainstem perforating vessels be spared during surgical clip placement.

Unfortunately, there are occasions when the size, shape and/or location of an aneurysm make both surgical clipping and endovascular embolization not possible for a particular patient. Generally, the prognosis for such patients is not good.

A significant advance in art of endovascular aneurysm occlusion is described in International Publication Number WO 99/40873, published August 19, 1999 and International Publication Number WO 00/47134, published August 12, 2000 [both naming Marotta et al.]. The Marotta device is highly advantageous since it can be navigated to the site of "hard to reach" aneurysms where blockage of the aneurysmal opening may be achieved resulting in obliteration of the aneurysm.

Despite this significant advance in the art, there is still room for improvement. For example, the Marotta device comprises a so-called "leaf portion" for blockage of the aneurysmal opening. Once properly aligned, the leaf portion is advantageously useful to occlude the aneurysm. However, delivery can be difficult when using conventional balloon dilation catheters, since these catheters are typically used in delivering stents which do not require a specific orientation of the stent in relation to the target body passageway. Further difficulties can be encountered when attempting to deliver and properly orient the Marotta device to a bifurcated body passageway.

Accordingly, it would desirable to have a catheter adapted to deliver and orient an endovascular prosthesis in a body passageway.

### DISCLOSURE OF THE INVENTION

Accordingly, in one of its aspects, the present invention provides a catheter for endovascular delivery of a device, the catheter comprising:
a first tubular member disposed in a proximal portion of the catheter and a second tubular member disposed in a distal portion of the catheter, the first tubular member and the second tubular member being rotatable with respect to one another, the second tubular member comprising a device deployment element disposed distally thereof; and
a torque member connected to the second tubular member and rotatable with respect to the first tubular member to cause rotation of the second tubular member with respect to the first tubular member.

In another of its aspects, the present invention provides a catheterization kit comprising:
a guide catheter;
a guidewire; and
a device delivery catheter comprising: a first tubular member disposed in a proximal portion of the catheter and a second tubular member disposed in a distal portion of the catheter, the first tubular member and the second tubular member being rotatable with respect to one another, the second tubular member comprising a device deployment element disposed distally thereof; and a torque member connected to the second tubular member and rotatable with respect to the first tubular member to cause rotation of the second tubular member with respect to the first tubular member.

Thus, the present inventors have discovered a catheter which may be used advantageously to deliver an endovascular prosthesis to a target body passageway and orient the prosthesis with respect to the prosthesis. The present catheter is advantageous for delivery and orientation of an endovascular prosthesis such as the Marotta device referred to hereinabove. A feature of the present catheter is the presence of two tubular members which are rotatable with respect to one another. Relative rotation of the two tubular members is achieved by a torque member which is connected to the distal of the two tubular members and rotatable with the respect to the proximal of the two tubular members. The term "rotatable", when used in connection with the two tubular members is intended to have a broad meaning and is not particularly restricted provided that the two tubular members may be rotated with respect to each other relatively easier than a single, continuous tubular member. The nature of the torque member is not particularly restricted provided that it allows relatively easier torquing or twisting of the tubular members compared with a single, continuous tubular member. In one embodiment, this may be achieved by selecting the torque member to be a tube (e.g., a hypotube), a wire and the like.

### BRIEF DESCRIPTION OF THE DRAWINGS

Embodiments of the present invention will be described with reference to the accompanying drawings, in which:
Figure 1 illustrates a perspective view of a first embodiment of the present catheter;
Figure 2 illustrates an enlarged view, in partial section, of portion of the catheter illustrated in Figure 1;
Figure 3 illustrates torque of the catheter illustrated in Figure 2 having an endovascular prosthesis mounted thereon;
Figure 4 illustrates a perspective view of a second embodiment of the present catheter;
Figure 5 illustrates an enlarged view, in partial section, of portion of the catheter illustrated in Figure 4;
Figure 6 illustrates an enlarged view of region A of Figure 2; and
Figure 7 illustrates use of the catheter of Figure 1 or Figure 4 to deliver and orient an endovascular prosthesis in a body passageway.

### BEST MODE FOR CARRYING OUT THE INVENTION

While various preferred embodiments of the present catheter will be described with reference to the Marotta endovascular prosthesis referred to hereinabove, this is for illustrative purposes only. Those of skill in the art will immediately recognize that the present catheter may be used to advantageously deliver and orient other endovascular prosthesis where it is desirable to orient the prosthesis in a particular manner.

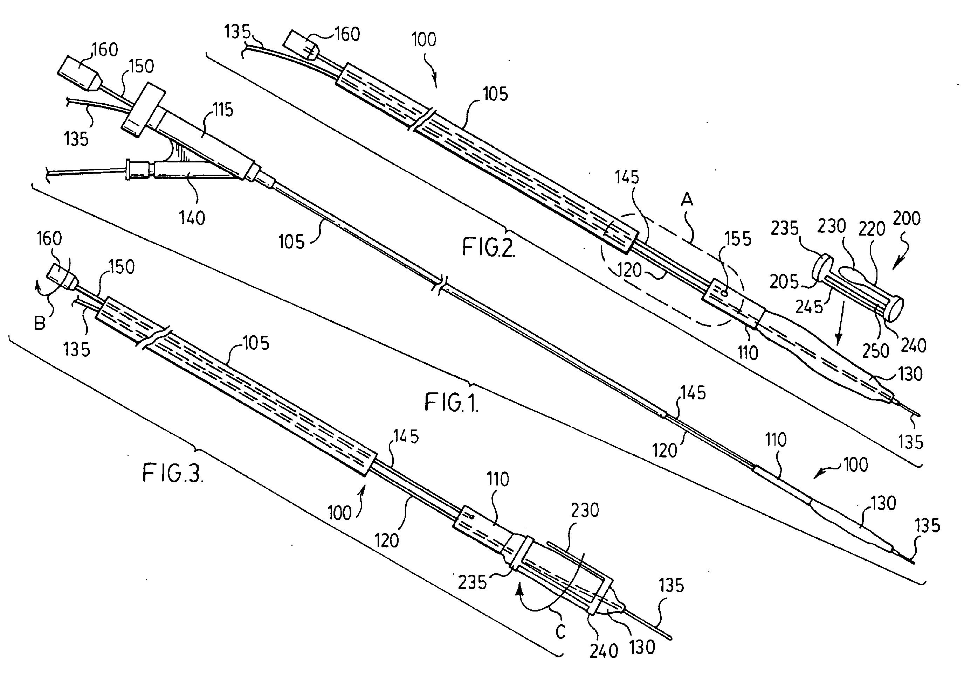

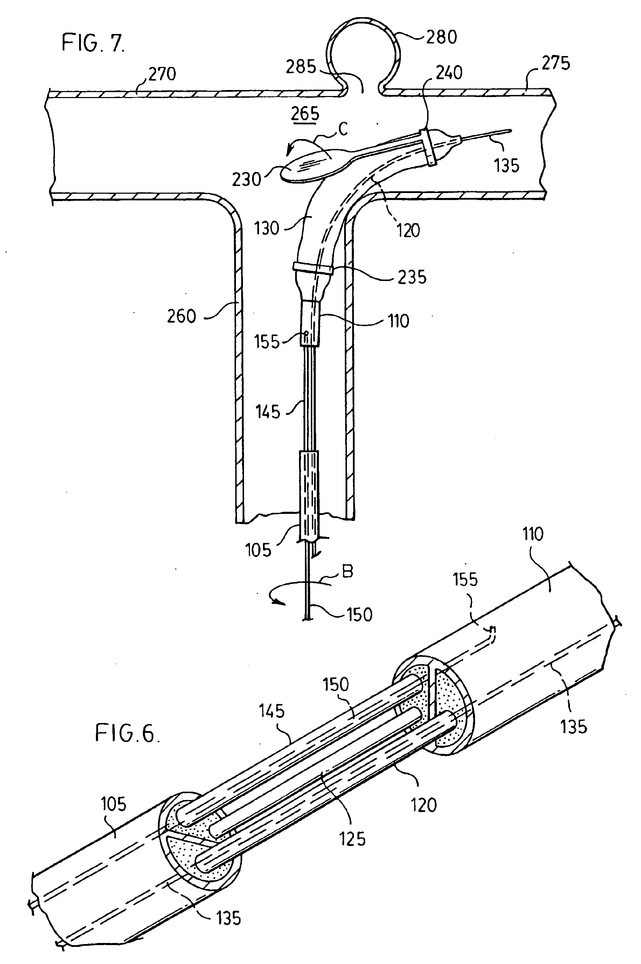

With reference to Figures 1-3 and 6, there is illustrated a balloon dilation catheter 100. Balloon catheter 100 comprises a first tubular member 105 and a second tubular member 110. Disposed at a proximal portion of first tubular member 105 is a Luer lock 115 or similar device. First tubular member 105 and second tubular member 110 are of similar design, each comprising a so-called "double-D" cross-section with each "D" comprising a passageway - see the can be seen particularly in Figure 6.

First tubular member 105 and second tubular member 115 are interconnected by a pair of lumen 120,125. As illustrated, lumen 120,125 serve to space apart first tubular member 105 and second tubular member 115. Preferably, the longitudinal spacing is less than about 10 cm, more preferably in the range of from about 1 cm to about 8 cm, most preferably in the range of from about 1 cm to about 5 cm. Lumen 120,125 are secured to first tubular member 105 and to second tubular member 110 by any suitable means (e.g., by an adhesive).

An expandable balloon 130 is secured to the distal end of second tubular member 110. The nature of balloon 130 and connection to second tubular member 110 is conventional and within the purview of a person skilled in the art.

Lumen 120 extends throughout first tubular member 105 through second tubular member 110 and emanates from balloon 130. The proximal end of lumen 120 exits from Luer lock 115 in a conventional manner. As illustrated, lumen 120 receives a guideline 135 which emanates from Luer lock 115 at the proximal end of catheter 100 and from balloon 130 at the distal end of catheter 100.

Lumen 125 extends through first tubular member 105, second tubular member 110 and comprises a distal opening (not shown) in communication with an interior of balloon 130. The proximal end of lumen 125 exits from an inflation port 140 of Luer lock 115 in a conventional manner. Thus, those of skill in the art will recognize the lumen 125 is a so-called inflation lumen used for inflation and deflation of balloon 130.

A further lumen 145 extends throughout first tubular member 105 and into a portion of second tubular member 110. A torque wire 150 is disposed in lumen 145 and is attached to second tubular member 110 at a connection point 155. The proximal end of lumen 145 exits from Luer lock 115 in a conventional manner. As illustrated torque 150 emanates from Luer lock 115. A torque handle 160 is connected to a proximal end of torque wire 150.

With reference to Figure 2 there is further illustrated an endovascular prosthesis 200 of similar construction as the Marotta device described hereinabove. Endovascular prosthesis 200 comprises a leaf portion 220 attached to a body 205. As illustrated, leaf portion 220 comprises a head 230. In the illustrated embodiment, head 230 of leaf portion 220 points away from distal end of prosthesis 200.

Body 205 further comprises a pair of rings 235,240 which are interconnected by a pair of struts 245,250. In the illustrated embodiment leaf portion 220 is connected to ring 240. Struts 245,250 preferably are dimensioned to confer to prosthesis 200 sufficient integrity while maximizing flexibility to provide enhanced navigation. The purpose of struts 245,250 is to interconnect rings 235,240 while allowing prosthesis 200 to be sufficiently flexible such that it can be navigated to the target body passageway yet be sufficiently expandable such that it can be fixed at the proper location in target body passageway. Struts 245,250 are not particularly important during expansion of prosthesis 200 (i.e., after the point in time at which prosthesis 200 is correctly positioned). Further, as will be apparent to those of skill in the art, leaf portion 220 is independently moveable with respect to proximal end and distal end of prosthesis 200 (in the illustrated embodiment, leaf portion 220 is independently moveable with respect to rings 235,240).

With reference to Figures 2 and 3, prosthesis 200 is mounted on balloon 130 of catheter 100 in a conventional manner. For example, rings 235,240 may be crimped on balloon 130 of catheter 100.

As will be appreciated by those of skill in the art, torquing or twisting handle 160 in the direction of arrow B (Figure 3) will result in twisting of wire 150 within Lumen 145. The applied torque is then conveyed to connection point 155 resulting in rotation of second tubular member 110 with respect to first tubular member 110. This results in rotation of prosthesis 200 in the direction of arrow C (Figure 3) facilitating orientation of head 230 in leaf portion 220.

With reference to Figure 7, delivery and deployment of prosthesis 200 mounted on balloon 130 of catheter 100 will be described.

Thus, there is illustrated a basilar artery 260 which terminates at a junction 265 which bifurcates into pair of secondary arteries 270,275. Interposed between junction 265 secondary artery 275 is an aneurysm 280. Aneurysm 280 has an opening 285 (shown enlarged for illustrative purposes only) through blood enters and sustains aneurysm 280.

Guidewire 135 is delivered secondary artery 275 in a conventional manner, for example using a guide catheter (not shown). Next catheter 100 having prosthesis 200 mounted on balloon 130 (Figure 3) is advanced over delivered guidewire 135. As balloon 130 approaches junction 265, the position of head 230 of leaf portion 220 is relative to opening 285 of aneurysm 280 is assessed (e.g., using X-ray radiography). If head 230 does not appear to be properly aligned with opening 285, wire 250 may be torqued in the direction of arrow B as described above resulting in rotation of second tubular member 110 with respect to first tubular member 105. This results in rotation of head 230 in the direction of arrow C until it is properly aligned with opening 285 of aneurysm 280.

Once endovascular prosthesis 200 is in the correct position, balloon 130 is expanded thereby exerting radially outward forces on rings 235,240. Initially, this results in expansion of ring 235 against the wall of basilar artery 260 and expansion of ring 240 in secondary artery 275. As expansion of balloon 130 continues, a portion of balloon 130 urges against head 230 of leaf portion 220 resulting in urging of leaf portion 220 against the walls of secondary artery 275 in a manner which results in blocking of opening 285 of aneurysm 280.

Next, balloon 130 is deflated and, together with guidewire 130, withdrawn from endovascular prosthesis 200. Endovascular prosthesis 200 is secured in position by rings 235,240 being urged against the walls of basilar artery 260 and secondary artery 275, respectively. Further, leaf portion 220 is secured in position by a combination forces against it by the flow of the blood into junction 265 and the inherent forces upon flexure of body 205 to navigate the distal end of prosthesis 200 into secondary artery 275. Once leaf portion 220 blocks opening 285, aneurysm 280 is obliterated thereafter.

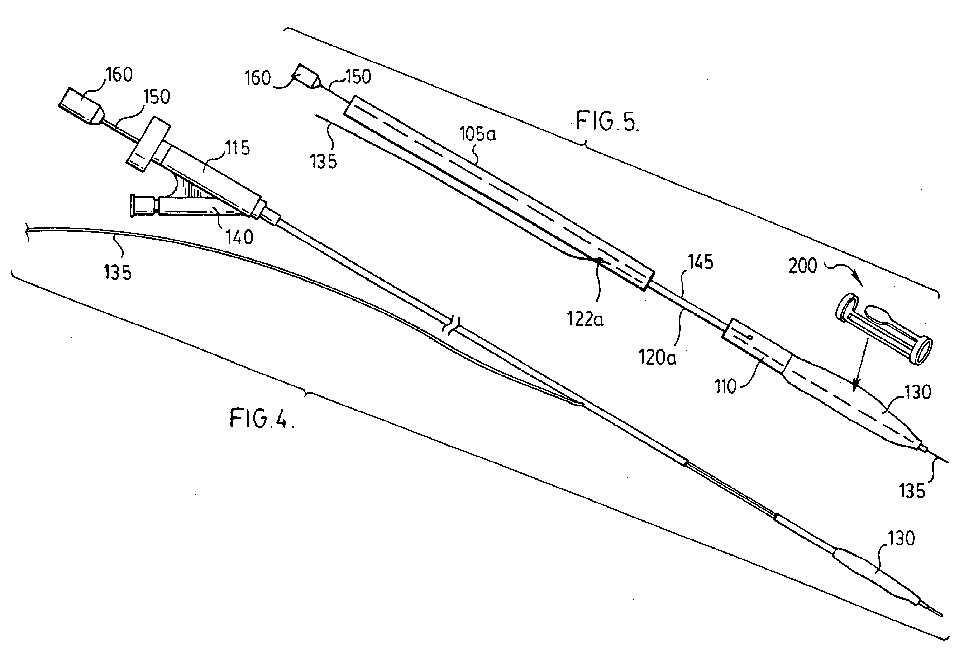

In the embodiment illustrated in Figures 1-3, is lumen 120 contains guidewire 135 in a so-called "over-the-wire" configuration. Figures 4 and 5 illustrate the present catheter in a so-called "mono-rail" configuration. In Figures 1-5, like numbers designate like elements. Modified elements in Figures 4 and 5 are designated with the suffix "a". Thus, in the catheter of Figures 4 and 5, first tubular member 105a has been modified to include an opening 122a through which guidewire 135 enters lumen 120a. The use of the catheter illustrated in Figures 4 and 5 is similar to that described above with the additional benefit of being able to rapidly exchange guidewire 135, if needed. See United States patent 4,748,982 [Horzewski et al.] and the references cited therein for a general discussion on "monorail" delivery systems and rapid exchange of guidewires using such a system.

While this invention has been described with reference to illustrative embodiments and examples, the description is not intended to be construed in a limiting sense. Thus, various modifications of the illustrative embodiments, as well as other embodiments of the invention, will be apparent to persons skilled in the art upon reference to this description. For example, while the illustrated embodiments depict a catheter having a balloon portion to expand the prosthesis (typically a balloon-expandable prosthesis such as a stainless steel stent), it is possible to modify the illustrated embodiments to replace the balloon portion with a retractable sheath thereby allowing the present catheter to facilitate deployment of a self-expandable endovascular prosthesis (e.g., a device made from a shape memory alloy and the like). It is therefore contemplated that the appended claims will cover any such modifications or embodiments.

## Claims

1. A catheter (100) for endovascular delivery of a device, the catheter (100) comprising:
a first tubular member (105) disposed in a proximal portion of the catheter (100) and a second tubular member (110) disposed in a distal portion of the catheter (100), the first tubular member (105) and the second tubular member (110) being rotatable with respect to one another, the second tubular member (110) comprising a device deployment element disposed distally thereof; and
a torque member (150) connected to the second tubular member (110) and rotatable with respect to the first tubular member (105) to cause rotation of the second tubular member (110) with respect to the first tubular member (105).

2. The catheter (100) defined in claim 1, wherein the device deployment element comprises an expandable member (130).

3. The catheter (100) defined in claim 1, wherein the device deployment element comprises a retractable sheath.

4. The catheter (100) defined in claims 1-3, wherein the torque member (150) comprises an elongate member.

5. The catheter (100) defined in any one of claims 1-4, wherein the torque member (150) is disposed in the first tubular member (105).

6. The catheter (100) defined in any one of claims 1-5, wherein the torque member (150) comprises a handle (160) near a proximal end thereof.

7. The catheter (100) defined in any one of claims 1-6, wherein the first tubular member (105) and the second tubular member (110) are spaced with respect to one another.

8. The catheter (100) defined in any one of claims 1-7, further comprising a sheath connected to the second tubular member (110) and rotatable with respect to the first tubular member (105), the sheath substantially covering the spacing between the first tubular member (105) and the second tubular member (110).

9. The catheter (100) defined in any one of claims 1-8, further comprising a first lumen (120) and a second lumen (125) disposed in each of the first tubular member (105) and the second tubular member (110), the first lumen (120) in communication with an interior of the expandable member (130) and the second lumen (125) for receiving a first guidewire (135), the second lumen (125) comprising an exit opening distal of the expandable member (130) to permit exit of the guidewire (135).

10. The catheter (100) defined in claim 9, wherein the second lumen (125) extends along a portion of the length of the first tubular member (105).

11. The catheter (100) defined in any one of claims 9-10, wherein the first tubular member (105) comprises an aperture through which the guidewire (135) may enter the second lumen (125).

12. The catheter (100) defined in any one of claims 9-11, wherein the second lumen (125) extends through a distal end of the device deployment element.

13. The catheter (100) defined in any one of claims 9-12, wherein each of the first tubular member (105) and the second tubular member (110) comprise a first passageway and a second passageway.

14. The catheter (100) defined in claim 13, wherein the first lumen (120) is disposed in the first passageway and the second lumen is disposed in the second passageway (125).

15. The catheter (100) defined in any one of claims 13-14, wherein the torque member (150) is disposed in the first passageway or the second passageway.

16. The catheter (100) defined in any one of claims 1-15, wherein the device deployment element (130) comprises a balloon portion.

17. The catheter (100) defined in any one of claims 1-16, further comprising an expandable endovascular prosthesis (200) mounted on the device deployment element.

18. The catheter (100) defined in claim 17, wherein the expandable prosthesis comprises a stent.

19. The catheter (100) defined in claim 17, wherein the expandable prosthesis comprises a stent graft.

20. The catheter (100) defined in claim 17, wherein the expandable prosthesis comprises a prosthesis for occlusion of an aneurysm.

21. A catheterization kit comprising:
a guide catheter;
a guidewire (135); and
a device delivery catheter (100) defined in any one of claims 1-20.

## Patentansprüche

1. Katheter (100) zur endovaskulären Zuführung einer Vorrichtung, wobei der Katheter (100) umfasst:
ein erstes röhrenförmiges Teil (105), das in einem proximalen Abschnitt des Katheters (100) angeordnet ist, und ein zweites röhrenförmiges Teil (110), das in einem distalen Abschnitt des Katheters (100) angeordnet ist, wobei das erste röhrenförmige Teil (105) und das zweite röhrenförmige Teil (110) bezüglich einander gedreht werden können, wobei das zweite röhrenförmige Teil (110) ein Vorrichtungsentfaltungselement umfasst, das distal davon angeordnet ist; und
ein Drehmomentteil (150), das mit dem zweiten röhrenförmigen Teil (110) verbunden ist und bezüglich des ersten röhrenförmigen Teils (105) drehbar ist, um die Drehung des zweiten röhrenförmigen Teils (110) bezüglich des ersten röhrenförmigen Teils (105) zu bewirken.

2. Katheter (100) nach Anspruch 1, wobei das Vorrichtungsentfaltungselement ein erweiterbares Teil (130) umfasst.

3. Katheter (100) nach Anspruch 1, wobei das Vorrichtungsentfaltungselement eine einziehbare Umhüllung umfasst.

4. Katheter (100) nach Anspruch 1-3, wobei das Drehmomentteil (150) ein lang gestrecktes Teil umfasst.

5. Katheter (100) nach einem der Ansprüche 1-4, wobei das Drehmomentteil (150) im ersten röhrenförmigen Teil (105) angeordnet ist.

6. Katheter (100) nach einem der Ansprüche 1-5, wobei das Drehmomentteil (150) einen Griff (160) in der Nähe eines proximalen Endes davon umfasst.

7. Katheter (100) nach einem der Ansprüche 1-6, wobei das erste röhrenförmige Teil (105) und das zweite röhrenförmige Teil (110) mit Abstand voneinander angeordnet sind.

8. Katheter (100) nach einem der Ansprüche 1-7, ferner umfassend eine Umhüllung, die mit dem zweiten röhrenförmigen Teil (110) verbunden ist und bezüglich des ersten röhrenförmigen Teils (105) drehbar ist, wobei die Umhüllung den Zwischenraum zwischen dem ersten röhrenförmigen Teil (105) und dem zweiten röhrenförmigen Teil (110) im Wesentlichen abdeckt.

9. Katheter (100) nach einem der Ansprüche 1-8, ferner umfassend ein erstes Lumen (120) und ein zweites Lumen (125), die jeweils im ersten röhrenförmigen Teil (105) und im zweiten röhrenförmigen Teil (110) angeordnet sind, wobei das erste Lumen (120) mit einem Inneren des erweiterbaren Teils (130) und dem zweiten Lumen (125) zum Aufnehmen eines ersten Führungsdrahtes (135) in Kommunikation ist, wobei das zweite Lumen (125) distal vom erweiterbaren Teil (130) eine Ausgangsöffnung umfasst, um das Austreten des Führungsdrahtes (135) zu erlauben.

10. Katheter (100) nach Anspruch 9, wobei sich das zweite Lumen (125) entlang einem Abschnitt der Länge des ersten röhrenförmigen Teils (105) erstreckt.

11. Katheter (100) nach einem der Ansprüche 9-10, wobei das erste röhrenförmige Teil (105) einen Durchlass umfasst, durch den der Führungsdraht (135) in das zweite Lumen (125) eintreten kann.

12. Katheter (100) nach einem der Ansprüche 9-11, wobei sich das zweite Lumen (125) durch ein distales Ende des Vorrichtungsentfaltungselements erstreckt.

13. Katheter (100) nach einem der Ansprüche 9-12, wobei das erste röhrenförmige Teil (105) und das zweite röhrenförmige Teil (110) jeweils einen ersten Durchgang und einen zweiten Durchgang umfassen.

14. Katheter (100) nach Anspruch 13, wobei das erste Lumen (120) im ersten Durchgang angeordnet ist und das zweite Lumen im zweiten Durchgang (125) angeordnet ist.

15. Katheter (100) nach einem der Ansprüche 13-14, wobei das Drehmomentteil (150) im ersten Durchgang oder im zweiten Durchgang angeordnet ist.

16. Katheter (100) nach einem der Ansprüche 1-15, wobei das Vorrichtungsentfaltungselement (130) einen Ballonabschnitt umfasst.

17. Katheter (100) nach einem der Ansprüche 1-16, ferner umfassend eine erweiterbare endovaskuläre Prothese (200), die auf dem Vorrichtungsentfaltungselement montiert ist.

18. Katheter (100) nach Anspruch 17, wobei die erweiterbare Prothese einen Stent umfasst.

19. Katheter (100) nach Anspruch 17, wobei die erweiterbare Prothese ein Stent-Transplantat umfasst.

20. Katheter (100) nach Anspruch 17, wobei die erweiterbare Prothese eine Prothese zur Okklusion eines Aneurysmas umfasst.

21. Katheterisierungs-Kit, umfassend:
einen Führungskatheter;
einen Führungsdraht (135); und
einen Katheter zur Zuführung einer Vorrichtung (100) nach einem der Ansprüche 1-20.

## Revendications

1. Cathéter (100) pour la distribution endovasculaire d'un dispositif, le cathéter (100) comprenant :
- un premier élément tubulaire (105) disposé dans une partie proximale du cathéter (100) et un deuxième élément tubulaire (110) disposé dans une partie distale du cathéter (100), le premier élément tubulaire (105) et le deuxième élément tubulaire (110) étant susceptibles de tourner l'un par rapport à l'autre, le deuxième élément tubulaire (110) comprenant un élément de déploiement de dispositif disposé de façon distale par rapport à celui-ci ; et
- un élément de couple (150) relié au deuxième élément tubulaire (110) et susceptible de tourner par rapport au premier élément tubulaire (105) pour provoquer une rotation du deuxième élément tubulaire (110) par rapport au premier élément tubulaire (105).

2. Cathéter (100) selon la revendication 1, dans lequel l'élément de déploiement de dispositif comprend un élément extensible (130).

3. Cathéter (100) selon la revendication 1, dans lequel l'élément de déploiement de dispositif comprend une gaine rétractable.

4. Cathéter (100) selon les revendications 1 à 3, dans lequel l'élément de couple (150) comprend un élément allongé.

5. Cathéter (100) selon l'une quelconque des revendications 1 à 4, dans lequel l'élément de couple (150) est disposé dans le premier élément tubulaire (105).

6. Cathéter (100) selon l'une quelconque des revendications 1 à 5, dans lequel l'élément de couple (150) comprend une poignée (160) à proximité d'une extrémité proximale de celui-ci.

7. Cathéter (100) selon l'une quelconque des revendications 1 à 6, dans lequel le premier élément tubulaire (105) et le deuxième élément tubulaire (110) sont espacés l'un de l'autre.

8. Cathéter (100) selon l'une quelconque des revendications 1 à 7, comprenant en outre une gaine reliée au deuxième élément tubulaire (110) et susceptible de tourner par rapport au premier élément tubulaire (105), la gaine recouvrant sensiblement l'espacement entre le premier élément tubulaire (105) et le deuxième élément tubulaire (110).

9. Cathéter (100) selon l'une quelconque des revendications 1 à 8, comprenant en outre une première lumière (120) et une deuxième lumière (125) disposées dans chacun des premier élément tubulaire (105) et deuxième élément tubulaire (110), la première lumière (120) communiquant avec l'intérieur de l'élément extensible (130) et la deuxième lumière (125) recevant un premier fil de guidage (135), la deuxième lumière (125) comprenant une ouverture de sortie à distance de l'élément extensible (130) pour permettre la sortie du fil de guidage (135).

10. Cathéter (100) selon la revendication 9, dans lequel la deuxième lumière (125) s'étend le long d'une partie de la longueur du premier élément tubulaire (105).

11. Cathéter (100) selon l'une quelconque des revendications 9 et 10, dans lequel le premier élément tubulaire (105) comprend une ouverture à travers laquelle le fil de guidage (135) peut pénétrer dans la deuxième lumière (125).

12. Cathéter (100) selon l'une quelconque des revendications 9 à 11, dans lequel la deuxième lumière (125) s'étend à travers une extrémité distale de l'élément de déploiement de dispositif.

13. Cathéter (100) selon l'une quelconque des revendications 9 à 12, dans lequel chacun du premier élément tubulaire (105) et du deuxième élément tubulaire (110) comprend un premier passage et un deuxième passage.

14. Cathéter (100) selon la revendication 13, dans lequel la première lumière (120) est disposée dans le premier passage et la deuxième lumière est disposée dans le deuxième passage (125).

15. Cathéter (100) selon l'une quelconque des revendications 13 et 14, dans lequel l'élément de couple (150) est disposé dans le premier passage ou le deuxième passage.

16. Cathéter (100) selon l'une quelconque des revendications 1 à 15, dans lequel l'élément de déploiement de dispositif (130) comprend une partie de ballonnet.

17. Cathéter (100) selon l'une quelconque des revendications 1 à 16, comprenant en outre une prothèse endovasculaire extensible (200) montée sur l'élément de déploiement de dispositif.

18. Cathéter (100) selon la revendication 17, dans lequel la prothèse extensible comprend une endoprothèse vasculaire.

19. Cathéter (100) selon la revendication 17, dans lequel la prothèse extensible comprend une greffe d'endroprothèse vasculaire.

20. Cathéter (100) selon la revendication 17, dans lequel la prothèse extensible comprend une prothèse pour l'occlusion d'un anévrisme.

21. Trousse ou kit de cathétérisation comprenant :
- un cathéter de guidage ;
- un fil de guidage (135) ; et
- un cathéter de distribution de dispositif (100) selon l'une quelconque des revendications 1 à 20.
